(19) **Europäisches Patentamt / European Patent Office / Office européen des brevets**

(11) **EP 2 124 709 B1**

(12) **FASCICULE DE BREVET EUROPEEN**

(45) Date de publication et mention
de la délivrance du brevet:
**31.08.2016 Bulletin 2016/35**

(21) Numéro de dépôt: **08762153.8**

(22) Date de dépôt: **25.02.2008**

(51) Int Cl.:
**A61B 1/05** *(2006.01)*

(86) Numéro de dépôt international:
**PCT/FR2008/050312**

(87) Numéro de publication internationale:
**WO 2008/113957 (25.09.2008 Gazette 2008/39)**

(54) **DISPOSITIF D'ENDOSCOPE FLEXIBLE A ASSERVISSEMENT VISUEL**

FLEXIBLE ENDOSKOPVORRICHTUNG MIT VISUELLER KONTROLLE

FLEXIBLE ENDOSCOPE DEVICE WITH VISUAL CONTROL

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **23.02.2007 US 902862 P**

(43) Date de publication de la demande:
**02.12.2009 Bulletin 2009/49**

(73) Titulaires:
• **Université de Strasbourg
(Etablissement Public à Caractère Scientifique, Culturel et Professionnel)
67000 Strasbourg (FR)**
• **Centre National de la Recherche Scientifique
75794 Paris Cedex 16 (FR)**

(72) Inventeurs:
• **DE MATHELIN, Michel
67000 Strasbourg (FR)**
• **GANGLOFF, Jacques
67350 Mulhausen (FR)**
• **ZANNE, Philippe
67480 Roppenheim (FR)**
• **OTT, Laurent
67150 Limersheim (FR)**

(74) Mandataire: **Nuss, Laurent et al
Cabinet Nuss
10, rue Jacques Kablé
67080 Strasbourg Cedex (FR)**

(56) Documents cités:
WO-A-2006/005013    DE-C1- 19 529 950
US-A- 5 347 987    US-A- 5 906 578
US-A- 5 951 461    US-A1- 2004 097 789

EP 2 124 709 B1

**Description**

La présente invention concerne le domaine des équipements et des instruments à usage médical, plus particulièrement les équipements de salles d'opérations, de salles d'investigation médicales et de cabinets médicaux, et a pour objet un dispositif d'endoscope flexible à asservissement visuel et un procédé de stabilisation active d'un tel endoscope.

Les endoscopes flexibles sont bien connus et utilisés depuis de nombreuses années dans une pluralité d'applications médicales, aussi bien à des fins de diagnostics qu'à des fins opératoires ou d'assistance opératoire.

Chaque procédure nécessite généralement un endoscope flexible spécifique qui est adapté en conséquence et qui se distingue par sa longueur, son diamètre, la manoeuvrabilité de son extrémité invasive, ses accessoires, les outils ou instruments qu'il peut intégrer, ...

Toutefois, sur l'ensemble des endoscopes flexibles actuels, il est possible de mettre en évidence systématiquement au moins les trois parties constitutives suivantes :

- un moyen de commande manuel externe, généralement sous la forme d'une poignée de commande pourvue d'au moins un, préférentiellement deux, organes de commande sous forme de molettes ou analogues;

- un corps allongé souple et flexible, généralement à section circulaire et dans lequel sont ménagés plusieurs canaux longitudinaux pour le passage d'instruments ou de câbles de manoeuvre;

- une extrémité invasive fonctionnelle, notamment sous la forme d'un segment d'extrémité flexible du corps allongé (située à l'opposé de la poignée de commande), qui porte ou forme la tête de l'endoscope, est muni d'un système optique (par exemple du type caméra vidéo CCD ou faisceau de fibre optique) et qui peut être courbé ou fléchi selon au moins deux directions préférentiellement mutuellement perpendiculaires.

Les canaux du corps allongé s'étendent également à travers ce segment d'extrémité au niveau duquel ils débouchent.

L'actionnement de la tête de l'endoscope, c'est-à-dire l'angle de déflexion du segment d'extrémité, est contrôlé par des câbles de manoeuvre courant le long du corps allongé et reliés aux organes de commande.

Pour naviguer dans le corps du sujet, et ainsi contrôler la trajectoire de l'endoscope ou guider un ou plusieurs instruments vers une zone ou un organe cible, le praticien utilise les images fournies par le système optique, embarqué dans la partie active (caméra CCD, fibre optique). L'éclairage de la scène est également assuré par l'endoscope via des fibres optiques débouchant au niveau de l'extrémité fonnant tête. Le praticien agit activement sur l'endoscope en manipulant le corps allongé (translation et rotation) par él'intermédiaire de la poignée et contrôle positivement la flexion du segment d'extrémité dans au moins deux directions orthogonales.

Une fois sur le site opératoire, le praticien peut passer des instruments comme, par exemple, des pinces, des ciseaux, un crochet, un bistouri, une fibre laser ou encore une aiguille, selon le type d'intervention à réaliser, et les interchanger par la suite sans modifier la position de l'endoscope.

L'intérêt de l'utilisation des endoscopes flexibles susvisés a été mis en évidence récemment, de manière supplémentaire, dans le cadre d'une nouvelle technique chirurgicale mini invasive appelée NOTES (Natural Orifice Transluminal Endoscopie Surgery) [voir notamment ″Per-oral transgastric abdominal surgerv″, KO C.W., KALLOO A.N., Chinese Journal of Digestive Diseases 2006, 7 : 67-70] qui est actuellement en développement sur modèle animal, et au stade des premiers essais sur l'homme.

Elle consiste à accéder à la cavité abdominale en passant par un orifice naturel (bouche puis estomac, anus puis intestin, ...), puis à travers une membrane interne (paroi gastrique, intestinale, vaginale, ...) pour accomplir des traitements tels que la ligature des trompes de Fallope, une cholécystectomie ou bien encore une gastrojejunostomie. Une première opération sans incision cutanée sur patient humain est décrite dans la publication suivante : ″ Surgerv without scars : Report of transluminal cholecysstectomy in a human being″, J. Marescaux, B. Dallemagne, S. Perretta et al, Archives of Surgery, 2007, 142(9) : 823-826.

Les premiers essais en chirurgie transluminale ont été possibles par l'utilisation d'endoscopes flexibles conventionnels accompagnés d'outils endoscopiques qui servaient jusqu'alors aux diagnostics et aux traitements de pathologies présentes dans les voies gastriques. Ces instruments ne sont toutefois pas adaptés à ces nouvelles techniques.

Un des principaux problèmes rencontré lors de la mise en oeuvre de tels endoscopes flexibles provient du fait que la partie souple de l'endoscope n'est pas commandable de manière directe et absolue, du fait de l'absence de liaison rigide. Sa forme et sa position sont définies par les contraintes anatomiques (oe sophage, estomac), c'est-à-dire par les structures anatomiques avec lesquelles l'endoscope est en contact et par les effets de la gravité.

Une fois que l'endoscope a pénétré dans le corps du sujet, sa forme est inconnue du praticien. Ce dernier doit par exemple prendre appui sur les structures anatomiques environnantes pour guider l'endoscope, ces structures étant souvent elles-mêmes non fixes.

Une fois la zone cible atteinte, le praticien doit combiner différentes actions pour réaliser les mouvements souhaités.

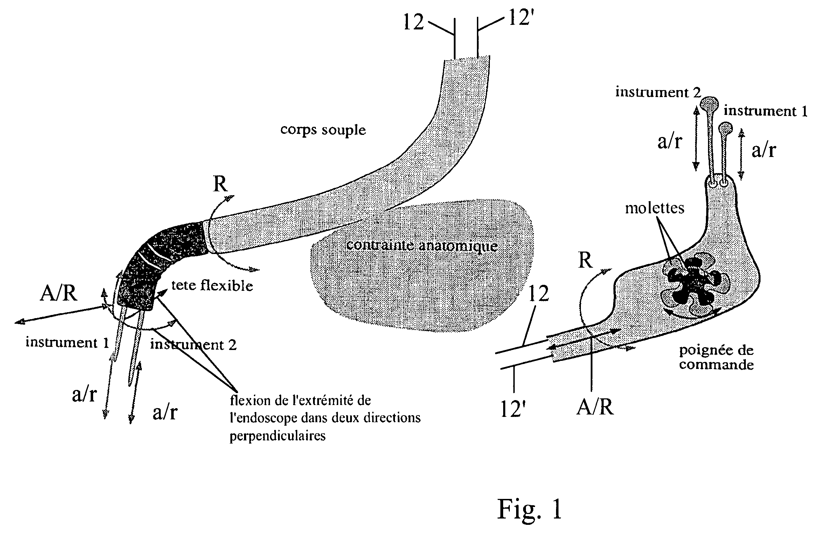

Comme l'illustre la figure 1, les actions possibles sont la rotation des molettes, l'avance/recul A/R de l'endoscope dans une direction dépendant de la position complète du corps de l'endoscope et la rotation R du corps de l'endoscope. La position de l'endoscope dans le corps du patient étant inconnue, les mouvements du corps de l'endoscope ont des effets difficilement prévisibles au niveau de l'image. De plus, la rotation des molettes produit une action certes maîtrisable, mais peu intuitive, qui nécessite un apprentissage long et délicat du praticien.

Les mouvements physiologiques des organes et du patient (respiration, mouvements du corps) sont des sources de perturbations sur l'endoscope flexible. La compensation de ces perturbations nécessite une coordination entre vision et mouvement de l'endoscope très complexe. Une bonne précision requiert donc un entraînement poussé du praticien.

En outre, la plupart des instruments endoscopiques actuels ne disposent pas d'articulations. Le praticien peut uniquement leur donner un mouvement d'avance/recul a/r indépendant du mouvement de l'endoscope. Pour leur donner une mobilité autre que dans l'axe de la caméra, le praticien doit déplacer la tête de l'endoscope. Ainsi, il n'est pas possible avec les instruments endoscopiques actuels d'effectuer des mouvements indépendants avec chacun desdits instruments.

Dans certains types d'interventions, le nombre de mobilités à gérer simultanément pour réaliser une intervention nécessite dans la pratique l'action de plusieurs praticiens sur le système endoscopique. Ces praticiens doivent alors se partager un espace de travail réduit autour de la poignée de commande de l'endoscope et faire preuve d'une bonne coordination pour réaliser les gestes médicaux ou chirurgicaux.

La complexité des manipulations et les mobilités réduites des outils ne permettent pas aux praticiens d'effectuer une opération aussi rapidement et avec la même dextérité qu'avec des techniques opératoires plus conventionnelles.

Le but de la présente invention consiste à proposer une solution permettant de s'affranchir des mouvements physiologiques, et plus généralement, des perturbations engendrées par l'environnement de l'endoscope, et de faciliter le contrôle précis de ce dernier par le praticien, en le soulageant de certaines tâches de commande et de contrôle, et ce sans modifier la constitution interne de l'endoscope, ni son mode de fonctionnement.

L'invention devrait également permettre une utilisation précise d'un endoscope flexible par des praticiens moins expérimentés et/ou moins performants et, en outre, améliorer de manière générale sa facilité de manipulation.

Par les documents FR-A-2 740 668, WO-A-9201414 et US-A-4 941 454, des endoscopes motorisés ont déjà été proposés. Toutefois, ces solutions connues ne permettent pas de répondre au but que s'est fixé l'invention.

Enfin, le document US-5 906 578 divulgue un dispositif d'endoscope flexible présentant les caractéristiques du préambule de la revendication 1.

Néanmoins, ce dispositif connu présente une constitution complexe et spécifique du moyen de commande motorisé qui est totalement intégré à la structure de l'endoscope et limité dans les possibilités de mouvement de l'extrémité de l'endoscope.

De plus, la méthode de vérification de l'approche mise en oeuvre est empirique et non systématique.

Ces inconvénients sont surmontés par l'invention grâce aux caractéristiques de la partie caractérisante de la revendication 1 qui permettent, en combinaison avec les caractéristiques du préambule de cette revendication, d'atteindre le but fixé.

Ainsi, il est proposé un dispositif de positionnement automatique de la tête de l'endoscope, en l'absence d'intervention positive du praticien.

Pour cela, il est fait usage des images prises par le système optique de l'endoscope lui-même pour le contrôle du segment d'extrémité flexible, ce par l'intermédiaire d'une motorisation de la commande de ce segment en combinaison avec un asservissement visuel. Il est ainsi réalisé une liaison virtuelle entre la tête de l'endoscope et la structure anatomique d'intérêt et ce malgré les mouvements physiologiques, l'interaction des instruments avec l'environnement et le mouvement d'enfoncement/de rétractation manuel de l'endoscope.

En fait, la localisation initiale et grossière du segment d'extrémité est obtenue par manipulation de l'endoscope en translation et en rotation par le praticien, l'invention permettant un contrôle du positionnement fin dudit segment d'extrémité, plus précisément de son orientation et de sa focalisation sur la cible.

Le principe de base mis en oeuvre par l'invention consiste, préférentiellement de manière itérative, à estimer l'erreur de pointage de l'endoscope par rapport à une cible et d'élaborer une commande, en temps réel, pour faire baisser voire annuler cette erreur de pointage.

L'invention sera mieux comprise, grâce à la description ci-après, qui se rapporte à un mode de réalisation préféré, donné à titre d'exemple non limitatif, et expliqué avec référence aux dessins schématiques annexés, dans lesquels :

la figure 2 est une représentation schématique d'un dispositif d'endoscope flexible selon l'invention ;
la figure 3 est une représentation fonctionnelle schématique d'une boucle de commande par asservissement visuel avec utilisation d'indices visuels pouvant être mise en oeuvre par l'invention ;
la figure 4A est une représentation fonctionnelle schématique illustrant la mise en oeuvre d'un algorithme de suivi visuel par minimisation de deuxième ordre pouvant être mis en oeuvre dans le cadre de l'invention ;
la figure 4B est une représentation fonctionnelle schématique d'une boucle de stabilisation active du dispositif d'endoscope flexible pouvant être mise en oeuvre par l'invention, intégrant l'algorithme de suivi représenté à la figure 4A ;
la figure 5 est un schéma bloc d'un exemple de modélisation de la boucle d'asservissement des figures 3 et 4B ;
la figure 6 est une représentation en perspective du moyen de commande du dispositif d'endoscope selon l'invention, sous forme de poignée motorisée ;
la figure 7 est une représentation en perspective d'une poignée de commande d'un endoscope, dépourvue d'organe de commande manuel et motorisé ;
les figures 8A à 8C sont des représentations en perspective illustrant successivement le montage des différentes composantes formant le moyen de commande motorisé, à savoir respectivement la pièce de support et de solidarisation, le premier actionneur et le second actionneur (seule une composante est représentée par figure, la représentation des deux autres composantes étant omise sur chaque figure 8A à 8C, ce pour faciliter la perception de chaque composante), et,
la figure 9 est une vue de détail en perspective de la pièce de transmission associée au moteur fonnant le premier actionneur, représenté sur la figure 8B.

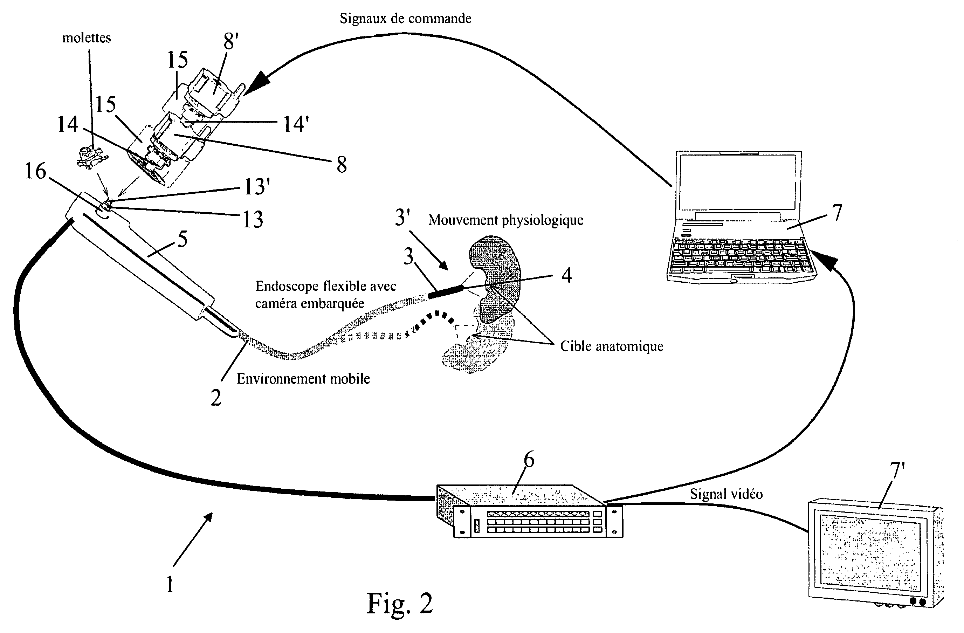

La figure 2 des dessins annexés montre un dispositif 1 d'endoscope flexible comprenant un corps 2 souple allongé présentant un segment d'extrémité 3 flexible portant ou fonnant la tête 3' de l'endoscope, muni d'un système optique 4 et pouvant être courbé ou fléchi selon au moins deux directions mutuellement perpendiculaires. Le corps allongé 2 étant relié fonctionnellement, à son autre extrémité, à un moyen de commande 5 apte à contrôler au moins les mouvements et/ou la disposition dudit segment d'extrémité 3.

Conformément à l'invention, le dispositif 1 comprend également des moyens d'orientation et de positionnement automatiques du segment d'extrémité 3 par asservissement visuel, ces moyens étant essentiellement constitués par un moyen de traitement vidéo 6 recevant les images ou signaux vidéo fourni(e)s par le système optique 4 de la tête de l'endoscope 3', par un moyen informatique 7 apte à réaliser des opérations d'asservissement visuel sur la base des signaux vidéo traités et fournissant des signaux de commande et par des moyens actionneurs 8, 8' faisant partie ou associés au moyen de commande 5 apte à contrôler le segment d'extrémité 3 (par flexion) et recevant les signaux de commande fournis par le moyen informatique 7.

Bien entendu, le moyen de traitement vidéo 6 peut constituer une unité séparée (Figure 2) ou éventuellement être intégré avec le moyen informatique 7 dans une même unité de traitement et de gestion.

En variante, il peut aussi être prévu qu'une partie du moyen de traitement vidéo 6, ou que certaines fonctions réalisées par ce dernier, soi(en)t localisée(s) dans le système optique 4.

Ce dernier peut également se présenter sous différentes variantes de réalisation, à savoir : une caméra vidéo embarquée dans l'endoscope au niveau du segment d'extrémité 3 du corps allongé souple 2 ; un faisceau de fibres optiques débouchant au niveau du segment d'extrémité 3 et relié à une caméra vidéo ou un dispositif analogue extérieur (fibroscope) ; un faisceau de microfibres optiques monté dans le corps 2 et relié à un dispositif d'exploitation extérieur (dispositif de fibroscopie cellulaire) ou analogue.

Ainsi, la tête 3' de l'endoscope peut soit comporter la totalité ou la partie active du système optique 4 transmettant des signaux vidéo, soit comporter seulement un élément passif d'acquisition des images, relié à une unité fonctionnelle déportée, le cas échéant extérieure à l'endoscope et éventuellement intégrée au moyen informatique 7.

De même, au moins un moyen 7' d'affichage de l'image vidéo fournie par le système optique 4 (par exemple caméra CCD) peut être prévu.

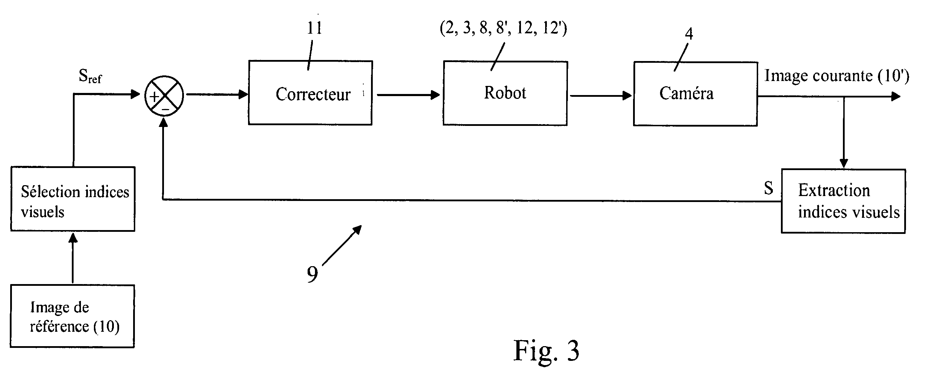

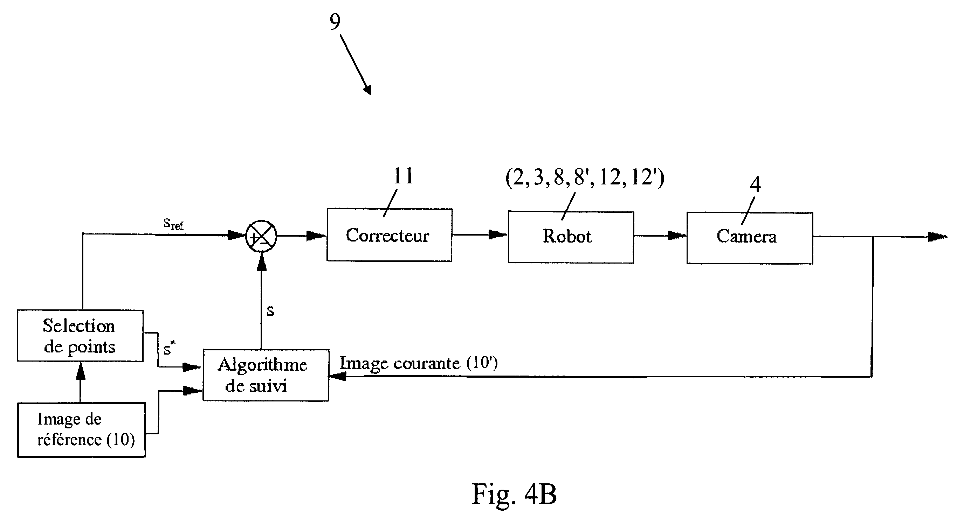

Selon l'invention, ressortant notamment des figures 3 et 4B des dessins annexés, les moyens 6, 7, 8, 8', 12, 12' de flexion et de positionnement automatique du segment d'extrémité 3 sont organisés fonctionnellement pour former au moins une boucle d'asservissement 9 destinée à minimiser l'erreur ou une mesure de dissemblance analogue entre, d'une part, un élément visuel ou d'information visuelle cible extrait d'une image de référence 10 et, d'autre part, un élément visuel ou d'information visuelle correspondant extrait de l'image courante 10' fournie par le système optique 4 de la tête de l'endoscope 3', ladite erreur étant exploitée pour délivrer des signaux de commande au(x) moyen(s) actionneur(s) 8, 8' contrôlant les mouvements et le positionnement dudit segment d'extrémité 3 selon au moins une direction.

Le moyen informatique 7 réalise par conséquent une commande numérique par asservissement visuel et renferme une modélisation logicielle du système physique complexe formé par l'endoscope, ces moyens de commande mécanique et sa motorisation, ladite modélisation traitant des signaux d'entrée formés par l'image de référence 10 et l'image courante 10' et fournissant en sortie des signaux de commande pour le ou les moyen(s) actionneur(s) 8, 8'.

Ainsi, pour obtenir une stabilisation active, on utilise les images fournies par le système optique 4 et exploitées sous forme d'images vidéo pour contrôler les déplacements de l'endoscope flexible, plus particulièrement de sa tête 3' (voir à ce sujet notamment : "A tutorial on visual servo control ", S. Hutchinson, G.D. Hager and P.I. Corke, IEEE

4

Transactions on Robotics and Automation, Vol. 12, 5, pp. 651-670, 1996).

La figure 3 représente de manière simplifiée une structure possible d'une boucle d'asservissement visuel 9 dont la mise en oeuvre est expliquée ci-dessous en relation avec le procédé selon l'invention.

Pour son fonctionnement, un tel asservissement visuel nécessite au préalable la détermination d'au moins un indice visuel dans l'image servant à la régulation.

Dans le cas d'un dispositif d'endoscope 1 à deux degrés de liberté, c'est-à-dire d'un endoscope dont le segment d'extrémité 3 peut être fléchi selon deux directions perpendiculaires, il est nécessaire de sélectionner deux indices visuels distincts ou un indice visuel à deux composantes (par exemple les coordonnée d'un point).

Or, l'environnement de travail dans lequel se situe la tête 3' de l'endoscope ne présente habituellement pas d'indices visuels particuliers tels que des points d'intérêts, des coins, des contours de formes rigides particulières, des lignes droites ou analogues, et ne permet donc pas directement l'application des procédés connus d'extraction, de coïncidence, de suivi et d'asservissement, sans mise en place de marqueurs additionnels dans l'environnement de travail.

Pour surmonter cette limitation, l'invention propose avantageusement que les éléments visuels ou d'information visuelle sont choisis dans le groupe formé par les motifs visuels, les réductions d'images et les parties ou zones d'images obtenues respectivement à partir de l'image de référence 10 et de l'image courante 10' fournie par le système optique 4, le cas échéant après traitement adapté de celles-ci.

En accord avec l'invention, le moyen informatique 7 est apte, par exécution d'un programme adapté, à déterminer en temps réel, c'est-à-dire au moins à la vitesse de fourniture des images vidéo, une approximation, préférentiellement par un traitement itératif, de la transformation à appliquer à l'image courante 10' aboutissant à une minimisation d'une fonction de coût entre une image réduite ou imagette T* obtenue à partir de l'image de référence 10 et une image réduite ou imagette T obtenue à partir de l'image courante 10' fournie par la tête 3' de l'endoscope et traitée par la transformation approximée courante.

Ainsi, les informations utiles à l'asservissement visuel s'appuient sur un algorithme du type "suivi visuel d'imagette de référence". La zone référence est apprise lors de la sélection manuelle, par le praticien, de la zone cible à stabiliser. Le suivi de cette zone est alors assuré en minimisant une mesure de dissemblance entre l'imagette de référence T* et l'imagette courante T.

Par image de "référence", on entend dans la présente l'image dans laquelle le praticien a identifié la cible anatomique. Elle est normalement constituée par une image initiale ou de base fournie par les moyens vidéo reliés à l'endoscope 2 ou éventuellement une image stockée, par exemple une image réelle de l'environnement anatomique de la cible acquise par sélection.

Selon une première variante de réalisation, l'algorithme de minimisation mis en oeuvre par le moyen informatique 7 réalise une minimisation paramétrique de l'erreur quadratique entre les éléments visuels ou d'information visuelle T* et T obtenus respectivement à partir de l'image de référence 10 et à partir de l'image courante 10', la transformation approximée étant une transformationde plan, par exemple, de type homographique.

Ainsi, l'algorithme de minimisation peut, par exemple, être dérivé d'un algorithme du type ESM, tel que décrit notamment dans la publication *"Improving vision-based control using efficient second-order minimization techniques "*, MALIS E., IEEE Int. Conf. on Rob. and Aut., Pages 1843-1848, 2004.

Un tel algorithme permet, comme indiqué, une minimisation paramétrique de l'erreur quadratique entre T et T*.

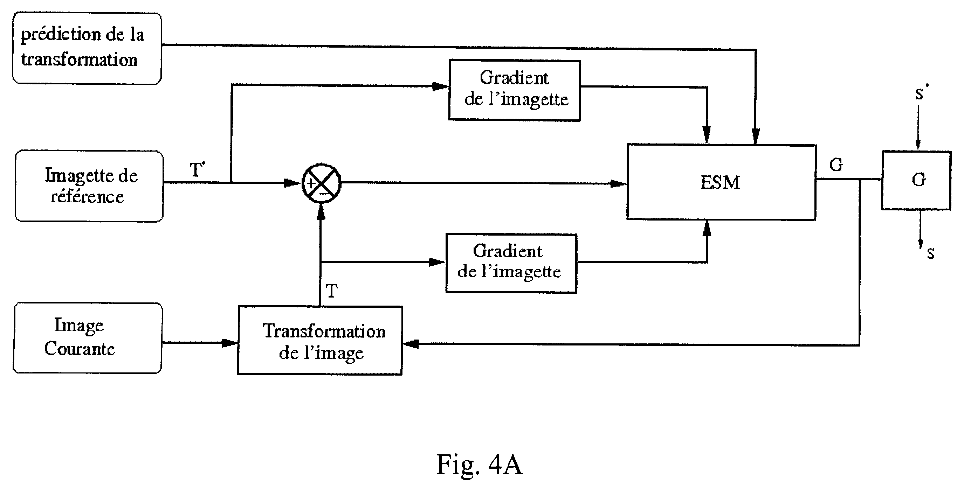

La configuration de mise en oeuvre de cet algorithme de minimisation est représentée sur la figure 4A et une description plus complète ressort de : "Real-time image-based tracking of planes using Efficient Second-order Minimization", S. Benhimane and E. Malis, IEEE/RSJ, Int. Conf. on Intelligent Robot and System, pages 943-948, 2004.

Dans ce contexte, la transformation estimée par le moyen informatique 7 est une transformation de plan, notamment homographique, permettant de localiser individuellement chaque pixel ou point de l'élément visuel et de l'utiliser comme indice visuel.

Pour réaliser une stabilisation de la tête de l'endoscope 3' en regard d'une cible anatomique mobile, la boucle 9 d'asservissement visuel telle que représentée sur la figure 4B, qui intègre l'algorithme selon la figure 4A, est proposée.

Selon une seconde variante de réalisation de l'invention, il peut aussi être prévu que l'algorithme de minimisation mis en oeuvre par le moyen informatique 7 réalise une minimisation statistique d'une mesure de dissemblance entre des histogrammes obtenus à partir des éléments visuels ou d'information visuelle T* et T obtenus respectivement à partir de l'image de référence 10 et à partir de l'image courante 10', la transformation approximée étant composée d'une translation dans le plan image et d'un zoom (réduction ou agrandissement).

Ainsi, en accord avec cette seconde variante, l'algorithme de minimisation peut être dérivé de celui connu sous la désignation algorithme du "mean-shift", tel que décrit par exemple dans : "Kermel-based object tracking", D. Comanicin et al., Patterne Analysis and Machine Intelligence, IEEE Transactions on, Volume 25, 5 mai 2003, pages : 564 à 577.

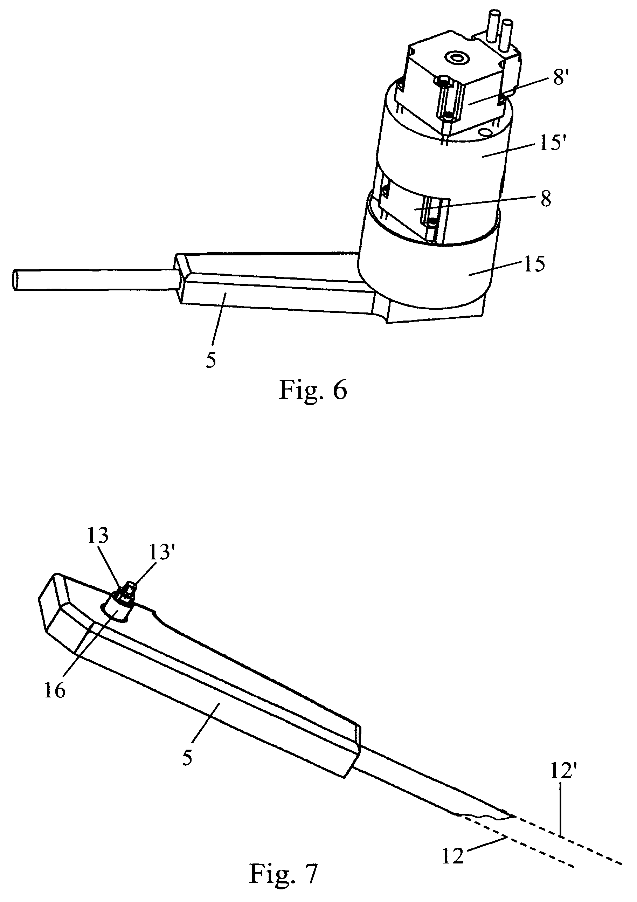

Comme l'illustre la figures 6 en relation avec un algorithme de suivi ou de minimisation précité (algorithme ESM précité ou algorithme du "mean shift"), le vecteur de la variable $S_{ref}$ préparamétrée contient les données image auxquelles la cible anatomique doit être stabiliser. Ce point est sélectionné initialement par le praticien sur l'image de référence 10 et peut être modifier dans l'image courante 10'. Le praticien spécifie également le point d'intérêt de la cible anatomique à stabiliser s* dans l'imagette T* de référence. Un des algorithmes de suivi précités est alors utilisé pour reconstruire la transformation entre l'imagette courante T et l'imagette de référence T*, cette transformation étant ensuite utilisée pour déterminer le vecteurs contenant les coordonnées du point à stabiliser dans l'image courante 10'.

Selon une caractéristique de l'invention, les moyens de positionnement automatique 6, 7, 8, 8' sont organisés en une boucle d'asservissement visuel bidimensionnel et comprennent deux actionneurs 8 et 8' indépendants contrôlant chacun les mouvements et le positionnement du segment d'extrémité 3 selon l'une des deux directions mutuellement perpendiculaires correspondant aux deux directions de l'image courante plane 10 fournie par la tête de l'endoscope 3'.

Eventuellement, une boucle d'asservissement 9 indépendante est prévue pour chaque moyen actionneur 8, 8', selon la nature du système.

Le contrôleur ou correcteur 11 faisant partie de la boucle d'asservissement 9 peut être choisi dans le groupe formé par les contrôleurs du type proportionnel, du type proportionnel/intégral/dérivé et du type prédictif ou répétitif.

Ainsi, lorsqu'il y a lieu de procéder à une compensation ou une annulation de mouvements périodiques, il est possible de mettre en oeuvre soit un contrôleur 11 du type répétitif tel que décrit par exemple dans : "Analysis and Synthesis of Discrete-Time Repetitive Controllers", M. Tomizuka et al., American Society of Mechanical Engineers Journal of Dynamic Systems, Measurement and Control, 111, 353-358, 1989; soit un contrôleur du type R-GPC tel que décrit par exemple dans : "Model Predictive Control for Pensation of Cyclic Organ Motions in Teleoperated Laparoscopic Surgery", J. Gangloff et al., Control Systems Technology, IEEE Transactions on, Volume14, Issue 2, mars 2006, pages : 235-246.

Toujours lorsque la perturbation est prédictible (par exemple dans le cas d'une respiration artificielle), le contrôleur 11 peut aussi intégrer un modèle de la perturbation, comme par exemple dans le contrôleur prédictif décrit dans la publication de E.F. Camacho, C Bordons, "Model Prédictive Control", ISBN 3-540-76241-8.

En accord avec l'invention, et comme cela ressort des figures 2, 6 et 8, le moyen de commande 5 comporte deux axes de commande 13 et 13' concentriques associés chacun à un câble de commande 12, 12' circulant le long du corps allongé 2 et relié au segment d'extrémité 3 pour contrôler la flexion ou le cintrage dudit segment 3 selon l'une des deux directions de flexion mutuellement perpendiculaires, l'axe extérieur 13 étant en relation d'entraînement avec un premier moyen actionneur 8 sous la forme d'un moteur à axe creux 8" par l'intermédiaire d'une pièce de transmission 14 dégagée dans une région centrale s'étendant dans la continuité de l'axe creux 8" dudit moteur 8, et l'axe intérieur 13' étant en relation d'entraînement, par l'intermédiaire d'une pièce d'entraînement 14' allongée, avec un second moyen actionneur 8' sous la forme d'un moteur aligné avec le premier moteur 8 suivant leurs axes de rotation, ladite pièce d'entraînement allongée 14' traversant le premier moteur 8 au niveau de son axe creux 8".

Avantageusement, les deux moteurs 8 et 8' sont assemblés mécaniquement entre eux pour former une unité de motorisation montée sur la poignée 5 formant le moyen de commande, par le biais d'une pièce 15 de support et de solidarisation, le cas échéant sous forme d'unité à raccordement mécanique amovible, autorisant ainsi une interchangeabilité avec les organes de commande manuelle (figure 2).

L'exemple de réalisation non limitatif de motorisation de la commande d'un endoscope flexible, illustré sur les figures 2 et 6 à 8, concerne plus particulièrement un endoscope du type 13801 PKS de la Société KARL STORZ.

La solution de motorisation proposée par l'invention permet de préserver la structure interne existante de l'endoscope, en préconisant le remplacement des molettes de commande manuelle par les actionneurs 8 et 8'.

Ces derniers se présentent par exemple sous la forme de moteurs à commande harmonique (par exemple du type FHA-8C) commandés par des servocontrôleurs à boucle de vitesse (par exemple du type Harmonic Drive SC-610). Lesdits moteurs sont reliés directement aux axes de commande 13 et 13', par liaison mécanique rigide et sans embrayage ni articulation, afin d'éviter au maximum tout jeu.

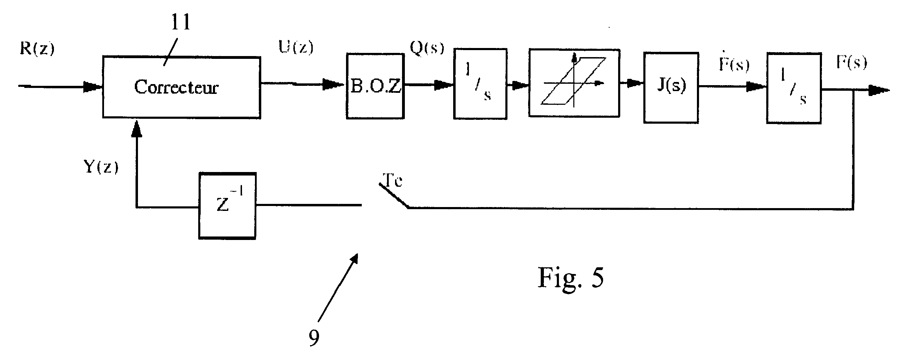

Un exemple de modélisation de la boucle d'asservissement visuel d'un tel système régulé et motorisé est représenté sous la forme d'un schéma bloc sur la figure 5 des dessins annexés.

En relation avec cette figure, on peut noter que le dispositif d'endoscope 1 étant un système mécanique à 2 degrés de liberté, les deux coordonnées x et y d'un indice visuel du type point dans l'image est une variable de rétroaction suffisante pour contrôler ledit système.

La vélocité de l'indice visuel F(s) dans l'image est reliée à la vélocité des actionneurs Q par une matrice d'interaction 2x2, que les inventeurs ont choisi d'estimer dans une phase préliminaire autour de la configuration de travail.

Le vecteur de vélocité des actionneurs Q(s)* est envoyé en tant que référence aux boucles de vitesse des amplificateurs de puissance commandant les moteurs des actionneurs 8 et 8'. La bande passante desdites boucles de vitesse étant nettement supérieure à la fréquence d'échantillonnage de la boucle d'asservissement visuel 9, la dynamique des actionneurs peut être négligée et par conséquent Q(s) est environ égal à Q(s)*.

Comme le montre la figure 5, la branche principale de la boucle comprend successivement les éléments suivants : correcteur / bloqueur d'ordre zéro B.O.Z./ intégrateur 1/s / module d'hystérésis (ensemble des jeux du dispositif 1) / matrice d'interaction J(s) / intégrateur 1/s et la branche de rétroaction comprend un élément de retard $Z^{-1}$ qui correspond aux temps d'acquisition des images.

La fonction de transfert temporel discrète du système représenté sous forme de schéma bloc sur la figure 5

est alors :

$$G(z) = \frac{Y(z)}{U(z)} = z^{-1}(1-z)Z\left\{\frac{J}{s^2}\right\} = J\frac{T_e.z^{-2}}{1-z^{-1}}$$

dans laquelle Z représente l'opérateur de la transformationen z.

Dans le cadre d'un exemple de réalisation mécanique pratique de l'invention, et en accord avec l'exemple de réalisation pratique illustré sur les figures 2 et 6 à 8 précitées, les deux axes de commande présentent des sections carrées et l'axe intérieur 13' s'étend de manière saillante à travers l'axe extérieur 13, ces deux axes 13 et 13' étant entourés d'une douille filetée 16 à section circulaire, à travers laquelle s'étendent ces deux axes 13 et 13' et qui est solidarisée de manière rigide à la structure de la poignée 5.

Une pièce support 15 creuse cylindrique, avantageusement solidarisée par vissage avec la douille 16 (par exemple par l'intermédiaire d'un orifice fileté venant en prise avec ladite douille) et reposant contre le boîtier de la poignée 5, réalise le montage des moteurs 8, 8' sur cette dernière, selon une configuration superposée.

Cette pièce 15 est en outre configurée de telle manière qu'elle assure un alignement axial des moteurs 8 et 8' avec les axes de commande concentriques 13 et 13'.

Cette pièce 15 supporte directement le moteur inférieur 8 et indirectement le moteur supérieur 8', ce dernier par l'intermédiaire d'une pièce support 15' additionnelle, solidarisée simultanément à ladite pièce 15 et audit moteur 8' (figures 2 et 6).

En variante au mode de solidarisation précité de la pièce support 15 avec la poignée 5, il peut également être prévu un mode de fixation par emboîtement amovible, autorisant une interchangeabilité aisée entre les molettes de commande manuelle et l'unité de motorisation 8, 8', 14, 14', 15, 15'.

La pièce 14 de transmission du mouvement entre le moteur inférieur 8 et l'axe de commande extérieur 13 présente une région centrale dégagée (selon la direction axiale des deux moteurs à l'état monté), et est muni au niveau de son fond d'une découpe carrée pour un engagement ajusté sur l'axe extérieur 13 et au niveau de sa partie supérieure de sites d'engagement avec la bride de sortie du moteur inférieur 8 à axe creux (par exemple liaison du type ergots/orifices).

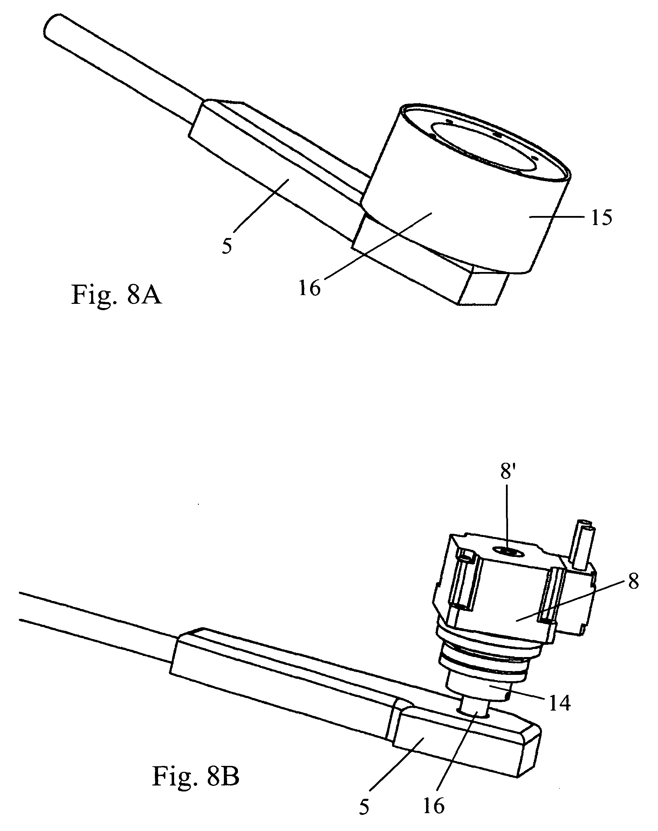

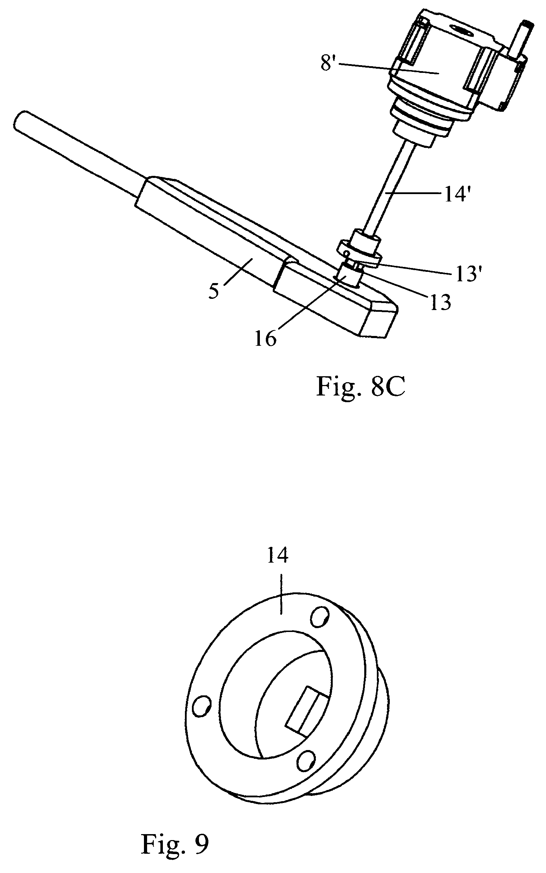

Comme le montre notamment la figure 8C, la pièce d'entraînement 14' assurant la transmission du mouvement entre le moteur supérieur 8' et l'axe intérieur 13', comporte une partie de raccordement inférieure (avec une ouverture à section carrée) destinée à venir en prise sur ledit axe 13' et une interface de raccordement supérieure en engagement avec la bride de sortie du moteur supérieur 8' (par exemple par l'intermédiaire d'une liaison ergots/orifices).

Le moteur supérieur 8' est maintenu en alignement axial avec le moteur inférieur 8 et les axes de commande 13 et 13' par l'intermédiaire de la pièce support additionnelle 15'.

Selon une variante non représentée et ne faisant pas partie de l'invention, les moyens actionneurs 8, 8' peuvent aussi se présenter sous la forme de moteurs directement logés dans la poignée formant le moyen de commande 5 et en relation d'entraînement chacun avec un câble de commande de la déformation par flexion du segment d'extrémité 3.

Dans le cadre de cette dernière variante, le moyen de commande 5 peut également comporter au moins un organe manipulable par le praticien, tel qu'une molette, une manette ou analogue, apte à commander au moins un actionneur 8, 8', ce de manière alternative ou superposée par rapport à la commande automatique par asservissement visuel, ledit ou lesdits organe(s) étant soi(en)t monté(s) au niveau de la poignée 5, soit déporté(s) par rapport à cette dernière.

Ainsi, il est possible de disposer d'une commande manuelle assistée, qui peut être superposée à la commande automatique par asservissement visuel ou fonctionner de manière découplée par rapport à cette dernière.

Dans le cas de la mise en oeuvre de molettes embarquées sur la poignée 5, le praticien retrouvera la configuration de manipulation actuelle, tout en bénéficiant des avantages de l'invention.

Un exemple de procédé de stabilisation d'un endoscope flexible par asservissement visuel est décrit ci-après.

Ce procédé met en oeuvre un dispositif 1 d'endoscope flexible comprenant essentiellement un corps souple allongé 2 présentant un segment d'extrémité flexible 3 portant ou fonnant la tête 3' de l'endoscope, muni d'un système optique 4 et pouvant être courbé ou fléchi selon au moins deux directions mutuellement perpendiculaires, ledit corps allongé 2 étant relié fonctionnellement, à son autre extrémité (opposée ou segment 3), à un moyen de commande 5 apte à contrôler au moins les mouvements et/ou la disposition dudit segment d'extrémité, plus particulièrement un dispositif tel que décrit précédemment.

Ce procédé consiste à réaliser automatiquement, par l'intermédiaire de moyens 6, 7, 8 et 8' de flexion et de positionnement automatiques du segment d'extrémité 3 organisé fonctionnellement en au moins une boucle d'asservissement 9, un traitement des images fournies par le système optique 4 de la tête 3' de l'endoscope, des opérations d'asservissement visuel sur la base des signaux vidéo traités et l'élaboration et la fourniture de signaux de commande à des moyens actionneurs 8, 8' faisant partie ou associés au moyen de commande 5.

Les opérations d'asservissement consistent essentiellement à minimiser l'erreur ou une mesure de dissemblance analogue entre, d'une part, un élément visuel ou d'information visuelle cible extrait d'une image de référence 10 et, d'autre part, un élément visuel ou d'information visuelle correspondant extrait de l'image courante 10' fournie par le système optique 4 de la tête de l'endoscope 3', ladite erreur étant exploitée pour délivrer des signaux de commande au(x) moyen(s) actionneur(s) 8, 8' contrôlant les mouvements et le positionnement dudit segment d'extrémité 3 selon au moins une direction.

Préférentiellement, les éléments visuels ou d'information visuelle sont choisis dans le groupe formé par les motifs visuels, les réductions d'image et les parties ou zones d'image obtenues respectivement à partir de l'image de référence 10 et de l'image courante 10' fournie par le système optique 4, le cas échéant après traitement adapté de celles-ci.

Conformément à un mode de réalisation avantageux, le procédé consiste, dans le cadres des opérations d'asservissement réalisées par le moyen informatique 7, à déterminer en temps réel, c'est-à-dire au moins à la vitesse de fourniture des images vidéo, une approximation, préférentiellement par un traitement itératif, de la transformation à appliquer à l'image courante 10' aboutissant à une minimisation d'une fonction de coût entre une image réduite ou imagette T* obtenue à partir de l'image de référence 10 et une image réduite ou imagette T obtenue à partir de l'image de référence 10' fournie par la tête 3' de l'endoscope et traitée par la transformation approximée courante.

Selon une première variante du procédé, ce dernier peut consister à mettre en oeuvre un algorithme de minimisation réalisant une minimisation paramétrique de l'erreur quadratique entre les éléments visuels ou d'information visuelle T* et T obtenus respectivement à partir de l'image de référence 10 et à partir de l'image courante 10', la transformation approximée étant une transformation de plan, par exemple, de type homo graphique.

Selon une seconde variante du procédé, ce dernier peut consister à mettre en oeuvre un algorithme de minimisation réalisant une minimisation statistique d'une mesure de dissemblance entre des histogrammes obtenus à partir des éléments visuels ou d'information visuelle T* et T obtenus respectivement à partir de l'image de référence 10 et à partir de l'image courante 10', la transformation approximée étant composée d'une translation dans le plan image et d'un zoom.

Avantageusement, il est prévu que les mouvements et le positionnement du segment d'extrémité 3' sont contrôlés par deux actionneurs indépendants 8 et 8' associés chacun à l'une de deux directions perpendiculaires correspondant à deux directions de l'image courante plane 10' fournie de manière répétée par la tête de l'endoscope 3'.

En relation avec la figure 3, la mise en oeuvre pratique du procédé selon l'invention peut comprendre deux phases successives, à savoir, une phase de paramétrage manuel d'une consigne $s_{ref}$ et une phase de régulation automatique à cadence vidéo de l'erreur : $e = s - s_{ref}$, où s est la valeur courante correspondant à la consigne, vers zéro.

La phase de paramétrage peut consister, pour le praticien, à déterminer la consigne $s_{ref}$ de la boucle d'asservissement 9, sous la forme d'indices visuels adéquats (par exemple coordonnées x, y d'un point) sélectionnés dans une image de référence 10, en vue de contrôler les différents degrés de liberté de l'endoscope flexible, en particulier de son extrémité libre 3.

Puis, la phase de régulation automatique peut consister à répéter de manière cyclique, les étapes suivantes :

a) extraction dans l'image courante 10' des indices visuels s courant correspondant aux indices visuel(s) $s_{ref}$ formant la consigne et sélectionnés dans l'image de référence 10 ;
b) calcul de l'erreur $e = s - s_{ref}$ ;
c) calcul des signaux de commande à appliquer aux actionneurs 8 et 8', notamment des signaux de vitesse, pour faire diminuer l'erreur e ;
d) envoi des signaux de commande ;
e) acquisition d'une nouvelle image courante 10' par l'intermédiaire du système optique et éventuel traitement de cette image ;
f) retour à l'étape a).

Afin de rendre l'asservissement plus robuste et de rendre plus efficace la détection de la cible, il peut en outre être prévu de mettre en place préalablement un repère artificiel à proximité immédiate de la cible anatomique.

Un exemple de mise en oeuvre pratique de l'invention, ainsi que les avantages procurés par celle-ci, sont décrits dans la publication suivante : "Problématique de l'assistance robotique à la chirurgie transluminale endoscopique", Actes de la conférence Surgetica 2007 ; Computer-aided médical interventions ; septembre 2007, Chambéry, France, dont le contenu est intégré à la présente par référence.

Bien entendu, l'invention n'est pas limitée au mode de réalisation décrit et représenté aux dessins annexés. Des modifications restent possibles, notamment du point de vue de la constitution des divers éléments ou par substitution d'équivalents techniques, sans sortir pour autant du domaine de protection defini par les revendications.

**Revendications**

1. Dispositif d'endoscope flexible,
ce dispositif (1) comprenant un corps (2) souple allongé présentant un segment d'extrémité (3) flexible portant ou formant la tête de l'endoscope, muni d'un système optique (4) et pouvant être courbé ou fléchi selon au moins deux directions mutuellement perpendiculaires, ledit corps allongé (2) étant relié fonctionnellement, à son autre extrémité, à un moyen de commande (5) apte à contrôler au moins les mouvements et/ou la disposition dudit segment d'extrémité,
ce dispositif (1) comprenant également des moyens d'orientation et de positionnement automatique du segment d'extrémité (3) par asservissement visuel, essentiellement constitués par un moyen de traitement vidéo (6) recevant les images ou signaux vidéo fourni(e)s par le système optique (4) de la tête de l'endoscope (3'), par un moyen informatique (7) apte à réaliser des opérations d'asservissement visuel sur la base des signaux vidéo traités et fournissant des signaux de commande et par des moyens actionneurs (8, 8') faisant partie ou associés au moyen de commande (5) apte à contrôler le segment d'extrémité (3) et recevant les signaux de commande fournis par le moyen informatique (7),
les moyens (6, 7, 8, 8', 12, 12') d'orientation et de positionnement automatique du segment d'extrémité (3) étant organisés fonctionnellement pour former au moins une boucle d'asservissement (9) destinée à minimiser l'erreur ou une mesure de dissemblance analogue entre, d'une part, un élément visuel ou d'information visuelle cible extrait d'une image de référence (10) et, d'autre part, un élément visuel ou d'information visuelle correspondant extrait de l'image courante (10') fournie par le système optique (4) de la tête de l'endoscope (3'), ladite erreur étant exploitée pour délivrer des signaux de commande au(x) moyen(s) actionneur(s) (8, 8') contrôlant les mouvements et le positionnement dudit segment d'extrémité (3) selon au moins une direction,
le moyen informatique (7) étant apte, par exécution d'un programme adapté, à déterminer en temps réel, c'est-à-dire au moins à la vitesse de fourniture des images vidéo, une approximation, préférentiellement par un traitement itératif, de la transformation à appliquer à l'image courante (10') de manière à approcher cette dernière de l'image de référence, les moyens de positionnement automatique (6, 7, 8, 8') étant organisés en une boucle d'asservissement visuel bidimensionnel et comprennent deux actionneurs (8 et 8') indépendants contrôlant chacun les mouvements et le positionnement du segment d'extrémité (3) selon l'une de deux directions mutuellement perpendiculaires correspondant à deux directions de l'image courante plane (10') fournie par la tête de l'endoscope (3'),
dispositif (1) **caractérisé**
**en ce que** le programme exécuté par le moyen informatique (7), dans le cadre des opérations d'asservissement, réalise l'approximation par minimisation d'une fonction de coût entre une image réduite ou imagette (T*) obtenue à partir de l'image de référence (10) et une image réduite ou imagette (T) obtenue à partir de l'image courante (10') fournie par la tête (3') de l'endoscope et traitée par la transformation approximée courante-et, les images réduites ou imagettes comprenant les éléments visuels ou d'information visuelle cibles extraits des images de référence et courante, et
**en ce que** le moyen de commande (5) comporte deux axes de commande (13 et 13') concentriques associés chacun à un câble de commande (12, 12') circulant le long du corps allongé (2) et relié au segment d'extrémité (3) pour contrôler la flexion ou le cintrage dudit segment (3) selon l'une des deux directions de flexion mutuellement perpendiculaires, l'axe extérieur (13) étant en relation d'entraînement avec un premier moyen actionneur (8) sous la forme d'un moteur à axe creux (8") par l'intermédiaire d'une pièce de transmission (14) dégagée dans une région centrale s'étendant dans la continuité de l'axe creux (8") dudit moteur (8), et l'axe intérieur (13') étant en relation d'entraînement, par l'intermédiaire d'une pièce d'entraînement (14') allongée, avec un second moyen actionneur (8') sous la forme d'un moteur aligné avec le premier moteur (8) suivant leurs axes de rotation, ladite pièce d'entraînement allongée (14') traversant le premier moteur (8) au niveau de son axe creux (8").

2. Dispositif selon la revendication 1, **caractérisé en ce que** les éléments visuels ou d'information visuelle sont choisis dans le groupe formé par les motifs visuels, les réductions d'images et les parties ou zones d'images obtenues respectivement à partir de l'image de référence (10), sous la forme d'une image initiale ou de base fournie par le système optique (4) ou d'une image réelle de l'environnement anatomique de la cible acquise par sélection, et de l'image courante (10') fournie par le système optique (4), le cas échéant après traitement adapté de celles-ci.

3. Dispositif selon la revendication 1 ou 2, **caractérisé en ce que** l'algorithme de minimisation mis en oeuvre par le moyen informatique (7) réalise une minimisation paramétrique de l'erreur quadratique entre les éléments visuels ou d'information visuelle (T* et T) obtenus respectivement à partir de l'image de référence (10) et à partir de l'image courante (10'), la transformation approximée étant une transformation de plan, par exemple, de type homographique.

4. Dispositif selon la revendication 1 ou 2, **caractérisé en ce que** l'algorithme de minimisation mis en oeuvre par le moyen informatique (7) réalise une minimisation statistique d'une mesure de dissemblance entre des histogrammes obtenus à partir des éléments visuels ou d'information visuelle (T* et T) obtenus respectivement à partir de l'image de référence (10) et à partir de l'image courante (10'), la transformation approximée étant composée d'une translation dans le plan image et d'un zoom.

**5.** Dispositif selon la revendication 1 à 4, **caractérisé en ce qu'**une boucle d'asservissement indépendante est prévue pour chaque moyen actionneur (8, 8').

**6.** Dispositif selon l'une quelconque des revendications 1 à 5, **caractérisé en ce que** le contrôleur ou correcteur (11) faisant partie de la boucle d'asservissement (9) est choisi dans le groupe formé par les contrôleurs du type proportionnel, du type proportionnel/intégral/dérivé et du type prédictif ou répétitif.

**7.** Dispositif selon l'une quelconque des revendications 1 à 6, **caractérisé en ce que** les deux moteurs (8 et 8') sont assemblés mécaniquement entre eux pour former une unité de motorisation montée sur la poignée (5) formant le moyen de commande, par le biais d'une pièce (15) de support et de solidarisation, le cas échéant sous forme d'unité à raccordement mécanique amovible.

**8.** Dispositif selon la revendication 7, **caractérisé en ce qu'**il comporte au moins un organe manipulable par le praticien, tel qu'une molette, une manette ou analogue, apte à commander au moins un actionneur (8, 8'), ce de manière alternative ou superposée par rapport à la commande automatique par asservissement visuel, ledit organe étant soit monté au niveau de la poignée (5), soit déporté par rapport à cette dernière.

## Patentansprüche

**1.** Flexible Endoskopvorrichtung,
wobei diese Vorrichtung (1) einen länglichen, nachgiebigen Körper (2) umfasst, der einen flexiblen Endabschnitt (3) aufweist, der den Kopf des Endoskops trägt oder bildet, der mit einem optischen System (4) ausgestattet ist und in mindestens zwei aufeinander senkrecht stehenden Richtungen gebogen oder gekrümmt werden kann, wobei der genannte längliche Körper (2) an seinem anderen Ende mit einem Bedienungsmittel (5) funktionell verbunden ist, das in der Lage ist, mindestens die Bewegungen und/oder die Anordnung des genannten Endabschnitts zu steuern, wobei diese Vorrichtung (1) ebenfalls automatische Orientierungs- und Positionierungsmittel für den Endabschnitt (3) durch visuelle Regelung umfasst, die im Wesentlichen aus einem Videoverarbeitungsmittel (6) bestehen, das die Videobilder oder -signale empfängt, die vom optischen System (4) des Endoskopkopfes (3') ausgegeben werden, aus einem Datenverarbeitungsmittel (7), das in der Lage ist, visuelle Regelvorgänge auf der Grundlage der verarbeiteten Videosignale auszuführen und Steuersignale ausgibt, und aus Aktuatormitteln (8, 8'), die Teil der Bedienungsmittel (5) sind oder ihnen zugeordnet sind und die in der Lage sind, den Endabschnitt (3) zu steuern, und die die Steuersignale empfangen, die von den Datenverarbeitungsmitteln (7) ausgegeben werden, wobei die automatischen Orientierungs- und Positionierungsmittel (6, 7, 8, 8', 12, 12') für den Endabschnitt (3) derart funktionell organisiert sind, dass sie mindestens eine Regelschleife (9) bilden, die dazu dient, Fehler oder ein analoges Abweichungsmaß zwischen einerseits einem visuellen Zielelement oder einem Zielelement visueller Information zu minimieren, das einem Referenzbild (10) entnommen wurde, und andererseits einem entsprechenden visuellen Element oder Element visueller Information, das dem laufenden Bild (10') entnommen wurde, das vom optischen System (4) des Endoskopkopfes (3') stammt, wobei der genannte Fehler verwendet wird, um Steuersignale an das/die Aktuatormittel (8, 8') auszugeben, die die Bewegungen und die Positionierung des genannten Endabschnittes (3) in mindestens einer Richtung steuern,
wobei das Datenverarbeitungsmittel (7) in der Lage ist, durch Ausführung eines entsprechenden Programms in Echtzeit, d.h. mindestens mit der Ausgabegeschwindigkeit der Videobilder, eine Approximation, vorzugsweise durch eine iterative Verarbeitung der auf das laufende Bild (10') anzuwendenden Transformation zu bestimmen, um dieses letztere dem Referenzbild anzunähern, wobei die automatischen Positionierungsmittel (6, 7, 8, 8') in einer zweidimensionalen visuellen Regelschleife organisiert sind und zwei unabhängige Aktuatormittel (8, 8') umfassen, die jeweils die Bewegungen und die Positionierung des Endabschnitts (3) in einer der beiden aufeinander senkrecht stehenden Richtungen, die zwei Richtungen des ebenen laufenden Bildes (10') entsprechen, das vom Endoskopkopf (3') ausgegeben wird, steuern,
Vorrichtung (1), **dadurch gekennzeichnet, dass**
das vom Datenverarbeitungsmittel (7) im Rahmen der Regelvorgänge ausgeführte Programm die Approximation durch Minimierung einer Kostenfunktion zwischen einem reduzierten Bild oder Bildchen (T*), das aus dem Referenzbild (10) gewonnen wurde, und einem reduzierten Bild oder Bildchen (T), das aus dem laufenden Bild (10')

EP 2 124 709 B1

gewonnen wurde, das vom Endoskopkopf (3') ausgegeben wurde und durch die laufende approximierte Transformation verarbeitet wurde, ausführt, wobei die reduzierten Bilder oder Bildchen die visuellen Zielelemente oder Zielelemente visueller Information enthalten, die dem Referenzbild und dem laufenden Bild entnommen wurden, und dadurch, dass das Bedienungsmittel (5) zwei konzentrische Steuerachsen (13 und 13') aufweist, die jeweils mit einem Steuerkabel (12, 12') verbunden sind, das am länglichen Körper (2) entlangläuft und am Endabschnitt (3) befestigt ist, um die Biegung oder Krümmung des genannten Abschnitts (3) in einer der beiden aufeinander senkrecht stehenden Biegungsrichtungen zu steuern, wobei die äußere Achse (13) über ein Antriebsteil (14) mit einem ersten Aktuatormittel (8) in Form eines Motors mit Hohlachse (8") in Antriebsbeziehung steht, wobei das Antriebsteil in einem zentralen Bereich ausgebildet ist, der sich in der Fortsetzung der Hohlachse (8") des genannten Motors (8) erstreckt, und die innere Achse (13') über ein langgestrecktes Antriebsteil (14') mit einem zweiten Aktuatormittel (8') in Form eines Motors in Antriebsbeziehung steht, der mit dem ersten Motor (8) längs ihrer Drehachsen ausgerichtet ist, wobei das genannte langgestreckte Antriebsteil (14') den ersten Motor (8) im Bereich seiner Hohlachse (8") durchquert.

2. Vorrichtung nach Patentanspruch 1, **dadurch gekennzeichnet, dass** die visuellen Elemente oder Elemente visueller Information aus der Gruppe gewählt werden, die aus den visuellen Motiven, den Bildverkleinerungen und den Bereichen oder Abschnitten von Bildern, die aus dem Referenzbild (10) in Form eines Ausgangs- oder Basisbildes, das das optische System (4) ausgibt, oder einem realen, durch Auswahl gewonnenen Bild der anatomischen Umgebung des Ziels besteht, gewonnen wurden und aus dem laufenden Bild (10'), das aus dem optischen System (4) stammt, gegebenenfalls nach dessen entsprechender Verarbeitung.

3. Vorrichtung nach Patentanspruch 1 oder 2, **dadurch gekennzeichnet, dass** der vom Datenverarbeitungsmittel (7) ausgeführte Minimierungsalgorithmus eine parametrische Minimierung des quadratischen Fehlers zwischen den visuellen Elementen oder Elementen visueller Information (T* und T), die aus dem Referenzbild (10) bzw. aus dem laufenden Bild (10') stammen, ausführt, wobei die approximierte Transformation eine Ebenentransformation beispielsweise der Art einer Homographie ist.

4. Vorrichtung nach Patentanspruch 1 oder 2, **dadurch gekennzeichnet, dass** der vom Datenverarbeitungsmittel (7) angewandte Minimierungsalgorithmus eine statistische Minimierung eines Abweichungsmaßes zwischen aus den visuellen Elementen oder Elementen visueller Information (T* und T), die aus dem Referenzbild (10) bzw. aus dem laufenden Bild (10') stammen, erhaltenen Histogrammen ausführt, wobei die approximierte Transformation aus einer Translation in der Bildebene und einem Zoom besteht.

5. Vorrichtung nach Patentanspruch 1 bis 4, **dadurch gekennzeichnet, dass** für jedes Aktuatormittel (8, 8') eine unabhängige Regelschleife vorgesehen ist.

6. Vorrichtung nach irgendeinem der Patentansprüche 1 bis 5, **dadurch gekennzeichnet, dass** der Regler oder Korrektor (11), der der Regelschleife (9) angehört, aus der Gruppe, bestehend aus den Proportional reg lern, Proportional-Integral-Differential-Reglern und den prädiktiven oder repetitiven Reglern, ausgewählt wird.

7. Vorrichtung nach irgendeinem der Patentansprüche 1 bis 6, **dadurch gekennzeichnet, dass** die beiden Motoren (8 und 8') miteinander mechanisch zusammengesetzt sind, um eine Motorisierungseinheit zu bilden, die am Griff (5), der das Bedienungsmittel bildet, durch ein Trage- und Befestigungsstück (15), gegebenenfalls in Form einer Einheit zum abnehmbaren mechanischen Anschluss, montiert sind.

8. Vorrichtung nach Patentanspruch 7, **dadurch gekennzeichnet, dass** sie mindestens ein durch den Benutzer betätigbares Organ, wie etwa ein Rändelrad, einen Bedienungshebel oder Ähnliches, aufweist, das in der Lage ist, mindestens ein Aktuatormittel (8, 8') zu steuern, alternativ oder zusätzlich zur automatischen Steuerung durch visuelle Regelung, wobei das genannte Organ entweder im Bereich des Griffes (5) montiert ist oder von diesem entfernt.

**Claims**

1. Flexible endoscopic device,
said device (1) comprising an elongated flexible body (2) with a flexible end segment (3) that supports or forms the head of the endoscope, equipped with an optical system (4) and able to be curved or bent in at least two mutually perpendicular directions, said elongated body (2) being connected functionally at its other end to a control means (5) for monitoring at least the movements and/or the arrangement of said end segment,

said device (1) also comprising automatic means for orienting and positioning the end segment (3) by visual servoing, said means essentially consisting of a video processing device (6) receiving video images or signals supplied by the optical system (4) of the head of the endoscope (3') by a computer means (7) that can implement visual servo operations on the basis of processed video signals and providing control signals, and by actuating means (8, 8') that are part of or are associated with a control means (5) for monitoring the end segment (3) and receiving control signals supplied by the computer means (7),

the means (6, 7, 8, 8', 12, 12') for automatically orientating and positioning the end segment (3) being organised functionally to form at least one control loop (9) that is designed to minimise error or an analogous measure of dissimilarity between on the one hand a target visual element or visual information extracted from a reference image (10) and on the other hand a corresponding visual element or visual information extracted from the current image (10') supplied by the optical system (4) of the head of the endoscope (3'), said error being used to deliver control signals to the actuating means (8, 8') monitoring the movements and the positioning of said end segment (3) in at least one direction,

the computer means (7) being able, by executing a suitable program, to determine in real time, i.e. at least at the speed at which video images are supplied, an approximation, preferably by iterative processing, of the transformation to be applied to the current image (10') so as to approach the latter from the reference image,

the automatic positioning means (6, 7, 8, 8') being arranged in a bidimensional visual servo loop and comprising two independent actuators (8 and 8'), each controlling the movements and the positioning of the end segment (3) according to any one of two mutually perpendicular directions corresponding to two directions of the current plane image (10') provided by the head of the endoscope (3'),

the device (1) being **characterised in that**

the program executed by the computer means (7), within the context of servo operations, implements the approximation by minimising a cost function between a reduced image or thumbnail (T*) obtained from the reference image (10) and a reduced image or thumbnail (T) obtained from the current image (10') supplied by the head (3') of the endoscope and processed by the current approximated transformation,

the reduced images or thumbnail images comprising target visual elements or visual information extracted from reference and current images and

**in that** the control means (5) comprises two concentric control axes (13 and 13') each connected to a control cable (12, 12') circulating along the elongated body (2) and connected to the end segment (3) to control the bending or the bowing of said segment (3) according to one of two mutually perpendicular bending directions, the external axis (13) being drive-related to a first actuator (8) in the form of a motor with a hollow axis (8") by means of a disengaged transmission part (14) in a central area extending into the continuity of the hollow axis (8") of said motor (8) and the internal axis (13') being in drive relation, by means of an elongated drive part (14') with a second actuator (8') in the form a motor aligned with the first motor (8) according to their axes of rotation, said elongated drive part (14') traversing the first motor (8) at its hollow axis (8").

2. Device according to claim 1, **characterised in that** the visual elements or visual information are selected from a group formed by visual patterns, the reductions of images and parts or zones of images that are obtained respectively from the reference image (10), in the form of an initial image or a base image supplied by the optical system (4) or a real image of the anatomical environment of the target acquired by selection, and the current image (10') supplied by the optical system (4), if necessary after suitable processing of the latter.

3. Device according to claim 1 or 2, **characterised in that** the minimisation algorithm implemented by the computer means (7) produces a parametric minimisation of the quadratic error between the visual elements or visual information (T* and T) obtained respectively from the reference image (10) and from the current image (10'), whereby the approximated transformation is a plane transformation, for example, of the homographic type.

4. Device according to claim 1 or 2, **characterised in that** the minimisation algorithm implemented by the computer means (7) produces a statistical minimisation of a measure of dissimilarity between histograms that are obtained from visual elements or visual information (T* and T) obtained respectively from the reference image (10) and from the current image (10'), whereby the approximated transformation consists of a translation in the image plane and a zoom.

5. Device according to claim 1 to 4, **characterised in that** an independent servo loop is provided for each actuating means (8, 8').

6. Device according to any one of claims 1 to 5, **characterised in that** the monitor or corrector (11) that is part of the servo loop (9) is selected from a group formed by monitors of the proportional type, of the proportional/integral/derivative type, and of the predictive or repetitive type.

7. Device according to any one of claims 1 to 6, **characterised in that** the two motors (8 and 8') are assembled mechanically with one another to form a drive system unit that is mounted on the handle (5) that forms the control means, by means of a support and attaching part (15), if necessary in the form of a unit with a removable mechanical connection.

8. Device according to claim 7, **characterised in that** it comprises at least one element that can be manipulated by a practitioner, such as a roller, a lever or the like, able to control at least one actuator (8, 8') in an alternative or superimposed manner relative to the automatic command by visual servoing, whereby said element is either mounted on the handle (5) or offset relative to the latter.

Fig. 1

Fig. 2

molettes

15

8'

14

15

14'

8

16

13'

13

5

Signaux de commande

3' Mouvement physiologique

3

4

Endoscope flexible avec caméra embarquée

Cible anatomique

Environnement mobile

2

7

6

Signal vidéo

7'

1

EP 2 124 709 B1

EP 2 124 709 B1

$S_{ref}$

Sélection indices visuels

Image de référence (10)

11

Correcteur

(2, 3, 8, 8', 12, 12')

Robot

4

Caméra

Image courante (10')

S

Extraction indices visuels

9

Fig. 3

Fig. 4A

Fig. 4B

EP 2 124 709 B1

11

R(z)  U(z)  Q(s)  Ḟ(s)  F(s)

Correcteur  B.O.Z  1/s  J(s)  1/s

Y(z)

Z⁻¹

Tc

9

Fig. 5

Fig. 6

Fig. 7

Fig. 8A

5

16

15

Fig. 8B

8'

8

14

5

16

8'

14'

5

16

13'

13

Fig. 8C

14

Fig. 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2740668 A
- WO 9201414 A
- US 4941454 A
- US 5906578 A

**Littérature non-brevet citée dans la description**

- **KO C.W. ; KALLOO A.N.** Per-oral transgastric abdominal surgerv. *Chinese Journal of Digestive Diseases,* 2006, vol. 7, 67-70
- **J. MARESCAUX ; B. DALLEMAGNE ; S. PERRETTA et al.** Surgerv without scars : Report of transluminal cholecysstectomy in a human being. *Archives of Surgery,* 2007, vol. 142 (9), 823-826
- **S. HUTCHINSON ; G.D. HAGER ; P.I. CORKE.** A tutorial on visual servo control. *IEEE Transactions on Robotics and Automation,* 1996, vol. 12 (5), 651-670
- **MALIS E.** Improving vision-based control using efficient second-order minimization techniques. *IEEE Int. Conf. on Rob. and Aut.,* 2004, 1843-1848
- **S. BENHIMANE ; E. MALIS.** Real-time image-based tracking of planes using Efficient Second-order Minimization. *IEEE/RSJ, Int. Conf. on Intelligent Robot and System,* 2004, 943-948
- **D. COMANICIN et al.** Kermel-based object tracking. *Patterne Analysis and Machine Intelligence, IEEE Transactions on,* 05 Mai 2003, vol. 25, 564-577
- **M. TOMIZUKA et al.** Analysis and Synthesis of Discrete-Time Repetitive Controllers. *American Society of Mechanical Engineers Journal of Dynamic Systems, Measurement and Control,* 1989, vol. 111, 353-358
- **J. GANGLOFF et al.** Model Predictive Control for Pensation of Cyclic Organ Motions in Teleoperated Laparoscopic Surgery. *Control Systems Technology, IEEE Transactions on,* Mars 2006, vol. 14, 235-246
- **E.F. CAMACHO ; C BORDONS.** *Model Prédictive Control,* ISBN 3-540-76241-8
- Problématique de l'assistance robotique à la chirurgie transluminale endoscopique. *Actes de la conférence Surgetica,* 2007